(19) Europäisches Patentamt
European Patent Office
Office européen des brevets

(11) **EP 4 421 821 A1**

(12) # EUROPEAN PATENT APPLICATION

(43) Date of publication:
**28.08.2024 Bulletin 2024/35**

(21) Application number: **23305257.0**

(22) Date of filing: **27.02.2023**

(51) International Patent Classification (IPC):
**G16H 50/20** (2018.01)    **G16H 50/30** (2018.01)

(52) Cooperative Patent Classification (CPC):
**G16H 50/00; G16H 50/20; G16H 50/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International 94220 Charenton-Le-Pont (FR)**

(72) Inventors:
• **GAO, Yi**
  **138946 Singapore (SG)**
• **SPIEGEL, Daniel**
  **424796 Singapore (SG)**
• **DROBE, Bjorn**
  **339346 Singapore (SG)**

(74) Representative: **Korakis-Ménager, Sophie Essilor International Propriété Intellectuelle 147, rue de Paris 94220 Charenton-le-Pont (FR)**

(54) **CALCULATION MODULE, SYSTEM AND COMPUTER-IMPLEMENTED METHOD FOR DETERMINING AT LEAST ONE CAUSE OF A VISUAL FATIGUE OF A PATIENT**

(57)    Calculation module, system and computer-implemented method for determining at least one cause of a visual fatigue of a patient. The calculation module is configured to obtain values associated respectively with physiological parameters of the patient, to obtain causality coefficients associated respectively with the physiological parameters, to normalize the values to obtain normalized values associated respectively with the physiological parameters, to determine normalized scores associated respectively with the physiological parameters, based on the causality coefficients and the normalized values and to select at least one of the physiological parameters, using the normalized scores, as the at least one cause of the visual fatigue.

[Fig. 1]

**Description**

**Technical field**

This disclosure relates to devices and methods for determining the causes of visual fatigue of a patient.

**Background information and prior art**

Visual fatigue and the terms computer vision syndrome (CVS) and digital eye strain are often used interchangeably to describe different eye and vision-related problems that can result from a wide variety of causes, such as prolonged near work, extensive usage of digital devices (computers, tablets, smartphones, and e-readers), tear film quality, poor visual habits, poorly corrected, or uncorrected refractive errors.

The symptoms of visual fatigue may also be caused by poor lighting, glare on a digital screen, improper viewing distances, long work hours, alert levels, light spectrum, reduced contrast, small font, and uncorrected, undercorrected or poorly corrected refractive errors.

The methods of state of the art for visual fatigue assessment are classified as subjective or objective. Subjective assessments usually use questionnaires. Objective assessments usually use the results of tests realized by or on the patient. However, the methods of state of the art generally do not determine a cause of visual fatigue; they only quantify the overall amount of visual fatigue, usually followed by the prescription of one of the traditional anti-fatigue solutions such as blue-light filters and/or lenses with an addition used to near vision.

Thus, the goal of this disclosure is to provide methods and devices allowing the determination of the causes of visual fatigue and eventually determining a more accurate treatment to mitigate the visual fatigue.

**Summary**

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a calculation module for determining at least one cause of a visual fatigue of a patient. The calculation module is configured to obtain values associated respectively with physiological parameters of the patient, to obtain causality coefficients associated respectively with the physiological parameters, to normalize the values to obtain normalized values associated respectively with the physiological parameters, to determine normalized scores associated respectively with the physiological parameters, based on the causality coefficients and the normalized values and to select at least one of the physiological parameters, using the normalized scores, as the at least one cause of the visual fatigue.

Therefore, and compared to the state-of-the-art calculation modules that only consider visual fatigue as caused by insufficient accommodation, the calculation module of this disclosure allows a multi-factorial determination of the cause of the visual fatigue.

The calculation module of this disclosure may identify the real causes of visual fatigue for each patient according to their needs, lifestyle, work and entertainment habits and health conditions etc. Later, one can employ various bricks of anti-fatigue methods to personalize the solution to mitigate the visual fatigue of the patient.

Another aspect of this disclosure is a system for determining at least one cause of a visual fatigue of a patient, the system comprising a calculation module for determining at least one cause of a visual fatigue of a patient and a display unit. The calculation module is configured to obtain values associated respectively with physiological parameters of the patient, to obtain causality coefficients associated respectively with the physiological parameters, to normalize the values to obtain normalized values associated respectively with the physiological parameters, to determine normalized scores associated respectively with the physiological parameters, based on the causality coefficients and the normalized values, to select at least one of the physiological parameters, using the normalized scores, as the at least one cause of the visual fatigue and to command the display unit to display the at least one selected physiological parameter.

Another aspect of this disclosure is a computer implemented method for determining at least one cause of a visual fatigue of a patient. The computer implemented method comprises obtaining values associated respectively with physiological parameters of the patient, obtaining causality coefficients associated respectively with the physiological parameters, normalizing the values to obtain normalized values associated respectively to the values, determining normalized scores associated respectively with the physiological parameters, based on the causality coefficients and the normalized values and selecting at least one of the physiological parameters, using the normalized scores, as the at last one cause of the visual fatigue.

Another aspect of this disclosure is a computer program comprising instructions which, when the computer program is executed by a computer, cause the computer to carry out a method for determining at least one cause of a visual fatigue of a patient. The method comprises obtaining values associated respectively with physiological parameters of the patient, obtaining causality coefficients associated respectively with the physiological parameters, normalizing the values to obtain normalized values associated respectively to the values, determining normalized scores associated respectively with the physiological parameters, based on the causality coefficients and the normalized values and selecting at least one of the physiological parameters, using the normalized scores, as the at last one cause of the visual fatigue.

A computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a method for determining at least one cause of a visual fatigue of a patient. The method comprises obtaining values associated respectively with physiological parameters of the patient, obtaining causality coefficients associated respectively with the physiological parameters, normalizing the values to obtain normalized values associated respectively to the values, determining normalized scores associated respectively with the physiological parameters, based on the causality coefficients and the normalized values and selecting at least one of the physiological parameters, using the normalized scores, as the at last one cause of the visual fatigue.

## Description of the drawings

For a more complete understanding of the description provided herein and the advantages thereof, reference is now made to the brief descriptions below, taken in connection with the accompanying drawings and detailed description, wherein like reference numerals represent like parts.

Figure 1 represents the system for determining at least one cause of a visual fatigue of a patient.
Figure 2 represents a first example of the method for determining at least one cause of a visual fatigue of a patient.
Figure 3 represents a second example of the method for determining at least one cause of a visual fatigue of a patient.
Figure 4 represents an example of values of the physiological parameters of a patient.
Figure 5 represents a specific order of obtention of the values of the parameters.
Figure 6 represents an example of values of groups of physiological parameters of a patient.
Figure 7 represents another example of values of physiological parameters of a patient.

## Detailed description of embodiments

The detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### *Description of the system for determining a cause of a visual fatigue of a patient*

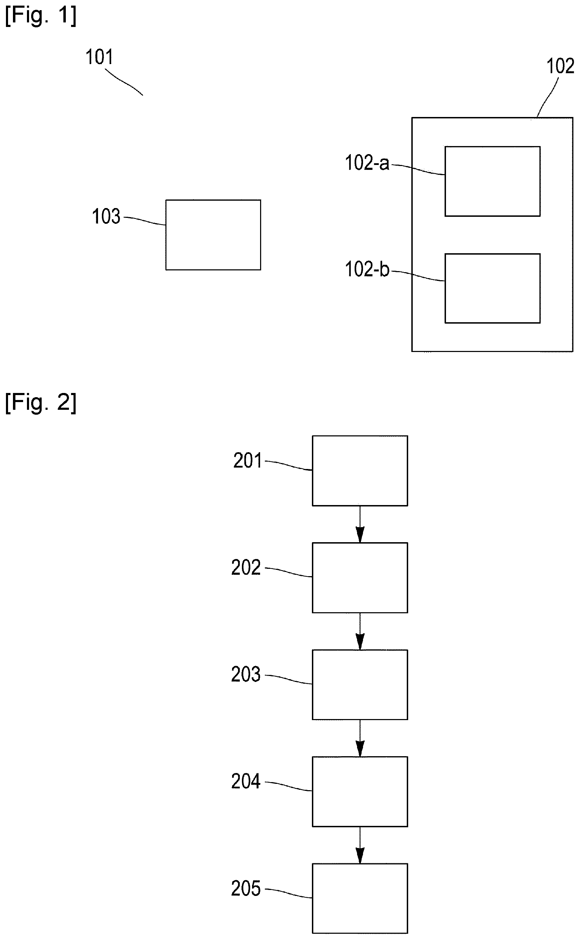

Figure 1 represents a system 101 for determining at least one cause of a visual fatigue of a patient. The system 101 comprises a calculation module 102 and a display unit 103.

The calculation module 102 comprises a memory 102-a and a processor 102-b.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

The memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

The calculation module 102 may be included in independent module, for example, a smartphone or a computer, a system on chip (SoC) or a graphics processing unit (GPU). The calculation module 102 may also be a virtual machine located on a cloud network or a server not co-located with the patient or the system 101.

The display unit 103 can be an electronic device or module for the visual presentation of data or images.

The system 101 can be for example a smartphone, in this case the display unit 103 is the screen of the smartphone and the calculation module 102 can be the system on chip of the smartphone.

The system 101 can be for example a computer, in this case the display unit 103 is the screen of the computer and the calculation module 102 can be constituted of the motherboard, the memory and the processor of the computer.

### *Description of the method for determining at least one cause of a visual fatigue of a patient*

To realize this determination of at least one case of a visual fatigue of a patient, the memory 102-a may store a computer program comprising instructions which, when the program is executed by the processor 102-b, cause the control module 102 to carry out a method, for example a computer implemented method, for determining at least one cause of a visual fatigue of a patient. The method as presented in figure 2 comprises:

- a step 201 of obtaining first values associated respectively with physiological parameters of the patient,
- a step 202 of obtaining first causality coefficients associated respectively with the physiological parameters,
- a step 203 of normalizing the first values to obtain first normalized values associated respectively with the physiological parameters,
- a step 204 of determining first normalized scores associated respectively with the physiological parameters, based on the first causality coefficients and the first normalized values,
- a step 205 of selecting at least one of the physiological parameters, using the first normalized scores, as the at last one physiological cause of the visual fatigue.

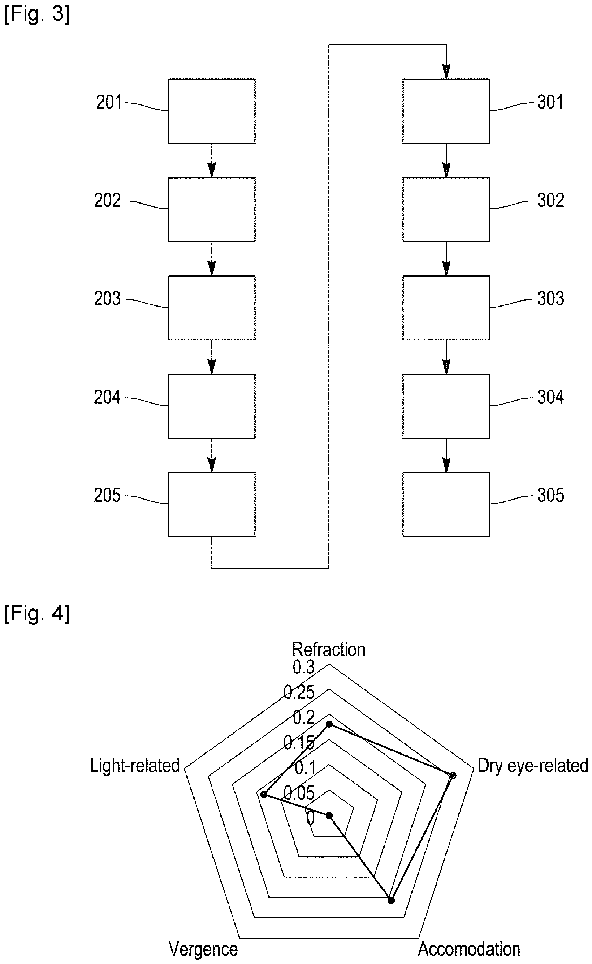

The figure 3 represents another embodiment of the method for determining the cause. In this embodiment in addition to physiological parameters of the patient, environmental parameters are taken into account.

In the embodiment of figure 3, in addition to the steps represented figure 2, the method for determining the at least one cause of the visual fatigue of the patient also comprises:

- a step 301 of obtain second values associated respectively with environmental parameters,
- a step 302 of obtaining second causality coefficients associated respectively with the environmental parameters,
- a step 303 of normalizing the second values to obtain second normalized values associated respectively with the environmental parameters
- a step 304 of determining second normalized scores associated respectively with the environmental parameters, by multiplying the second causality coefficients and the second normalized values,
- a step 305 of selecting one of the environmental parameters, using the second normalized scores, as an environmental cause of the visual fatigue.

Therefore, using the embodiment of the figure 3 one can determine a physiological cause of the visual fatigue and an environmental cause of the visual fatigue.

The method can also comprise a step of commanding the display unit to display the physiological cause (represented by the at least one selected physiological parameter) and/or the environmental cause (represented by the at least one selected environmental parameter). The method can also comprise a step of determining a treatment to mitigate the visual fatigue and a step of command the display unit to display the treatment. The treatment can depend on the physiological cause and/or the environmental cause.

The treatment may also vary from region to region, clinician to clinician, etc.

### *Description of the physiological parameters and the environmental parameters*

The physiological parameters can be chosen among:

- parameters related to a visual health of the patient,

  ◦ parameters related to a refraction of the patient (astigmatism, uncorrected ametropia, uncorrected presbyopia)
  ◦ parameters related to an abnormality of a vergence of the patient (vergence response abnormality, vergence amplitude abnormality

- parameters representing a dryness of an eye of the patient

  ◦ dry eye diagnosis
  ◦ parameters representing a stability of a tear film
  ◦ blink amplitude
  ◦ blink rate
  ◦ tear volume

- parameters related to an accommodation capacity of the patient

  ◦ amplitude of the accommodation
  ◦ accommodation facility

The environmental parameters can be chosen among:

- parameters characteristic of a light received by the patient,
- a luminance level of the light received by the patient,
- an amount of UV in the light received by the patient,
- a contrast between a luminance level of a light originated from an area of interest and a luminance level of an ambient light surrounding the area of interest,
- parameters characteristic an air surrounding the patient,
- a level of a humidity of the air surrounding the patient and
- parameters characteristic of a pollution of the air surrounding the patient.

The environmental parameters can also be chosen among parameters representing a glare on the screen or any working surface.
In other words, the physiological parameters and the environmental parameters may include dry eye, blinking patterns, tear film patterns, uncorrected refractive errors, accommodation- and vergence-related factors, blue light, glare, lighting environment, display content and settings, posture, etc., have been identified as causes of visual fatigue.
Some of the parameters can be grouped for example in the following manner

| Groups | Parameters |
| --- | --- |
| Refraction-related | Astigmatism |
| | Uncorrected ametropia |
| | Uncorrected presbyopia |
| Dry eye-related | Dry eye diagnosis |
| | Tear film stability (TBT) |
| | Blink amplitude |
| | Blink rate |
| | Tear volume |
| Accommodation-related | Amplitude |
| | Facility |
| Vergence | Vergence response abnormality |
| | Vergence amplitude abnormality |

(continued)

| Groups | Parameters |
|---|---|
| Light-related | Glare presence |
| | Blue light (BL) presence (e.g., no BL protection) |

The above set of parameters and groups is just an example and abbreviated version of the full picture of possible parameters causing visual fatigue. Other parameters and other groups can also be used to obtain a more precise determination of the cause of the visual fatigue.

In embodiments, after the steps 204 or 304, the normalized scores associated respectively with the parameters of the same group can be summed together or averaged, possibly with a weighted average. In this case during the steps 205 and 305 the group with the highest sum or average is selected as the cause of the visual fatigue.

The figure 4 represents an example of the values of the average of the different group for a theoretical subject whose visual fatigue problems are most likely related to dry eye, accommodation, and (uncorrected or not substantially) corrected refractive error.

### Description of the step 203 of normalizing the first values and the step 303 of normalizing the second values

Since the nature of the variety of parameters and their measurement units is very wide (for example refraction in diopters, tear film stability in seconds, or accommodative facility in cycles per minute), there is a need to normalize the scores to allow an accurate comparison.

Generally, the normalized values are comprised:

- between -100 and 100,
- between 0 and 100
- between -1 and 1 or
- between 0 and 1

The step 202 and 303 of normalization can be based on at least one of:

- a range of possible values of a parameter associated with the at least one of the values,
- a mean value of the range,
- a median value of the range
- a standard deviation of the range,
- a span of the range,
- a detection level of the parameter associated with the at least one of the values, and of
- a value representing a direction of an evolution of the physiological parameter associated with the at least one of the values.

The value representing the direction may be negative, for example -1, for parameters for which the higher the value, the less it affects the visual fatigue of the patient or maybe positive, for example 1, for the parameters for which the lower the value, the less it affects the visual fatigue of the patient.

In a first example, the normalization is realized by:

- comparing the at least one of the values with the detection level,

  when the at least one of the values is above the detection level, the normalized value is equal to a first predetermined value,

  when the at least one of the values is below the detection level, the normalized value is equal to a second predetermined value.

The first predetermined value may be for example 0, 1 or 100 and the second predetermined value may be for example -100, -1 or 0.

The first example is also called binary normalization.

In other words, in this first example the normalized values indicates that the parameter is likely to contribute to visual fatigue. For example, the presence of glare would result in a normalized value of 1.

For parameters with associated values that are a continuous variable one can realize this binary normalization by determining whether the value falls within a normality range of the values of this parameter. For example, a value of uncorrected astigmatism larger than 0.25 D would indicate that this parameter can affect visual fatigue therefore one can set the normalized value to 1.

In a second example, the normalization is realized by:

- subtracting the mean to the at least one of the values,
- dividing the subtraction by half the span, to obtain the normalized value.

The second example is also called normalization based on range of normality.

In this second example, for parameters with binary values, the normalization leads to the same results as the first example.

For parameters with continuous values, the range of normality (for example based on literature or on clinical experience) will be normalized between 0 and 1 whereby 1 indicates the bad end of the normality range and 0 indicates the good end of normality range. For example, an accommodative facility normally ranges between 10 and 20 cycles per minute with the general assumption that the more the better. Thus, a normalized value of 1 corresponds to 10 cycles per minute, and a normalized value of 0 corresponds to 20 cycles per minute.

In a third example the normalization is realized by:

- subtracting the mean to the at least one of the values,
- dividing the subtraction by the standard deviation of the range multiplied by a deviation factor to obtain the normalized value.

The deviation factor may be equal to 3. In this third example the normalized value can also be multiplied by the value representing the direction of the evolution of the physiological parameters. The equation to calculate the normalized score is then:

$$value_{[-1,1]} = direction * \frac{value_{measured} - value_{mean}}{value_{standard\ deviation} * 3}$$

Where:

- $value_{[-1,1]}$ is the normalized value between -1 and 1
- $value_{measured}$ is the measure value of the parameter
- $value_{mean}$ is the mean of the values of the parameter
- valuestandard deviation is the standard deviation of the values of the parameter
- $direction$ direction of the evolution of the parameter

For parameters with continuous values, one can also normalize the value with the mean and standard deviation in the specific population that each patient belongs to (determined by, not limited to, age, gender, and ethnicity).

The goal is to normalize values to be almost between -1 and 1 whereby -1 indicates the "good" end of the normality and 1 indicates the "bad" end of normality. *direction* parameter is -1 for values that the more the better, 1 for the less the better. Number 3 multiplying the standard deviation is chosen based on the fact that a normal distribution has more than 99% of the values within the difference of 3 standard deviations from the mean. For example, for the parameter Tear Film Stability, if the measured tear film break up time is 4 s or 9 s whereas the population mean is 5 s and the standard deviation is 2 s, and it the longer the better so direction parameter is -1. Then the normalized scores will be 0.17 and -0.67.

This normalized value can be further normalized to be between 0 and 1 by using the equation:

$$value_{[0,1]} = (value_{[-1,1]} + 1)/2$$

Where $value_{[-1,1]}$ is the normalized value between -1 and 1 and $value_{[0,1]}$ is the normalized value between 0 and 1.

In a nutshell to realize the normalization according to the third example one can:

- During a first step determine the direction parameter according to whether the outcome parameter is the more or the less the better.
- During a second step find the population mean and standard deviation from literature.
- During a third step calculate the normalized score using the above equation.

This equation normalizes scores to the range of -1 to 1. It can be further normalized to 0 to 1 if needed to be consistent with other factors by using $value_{[0,1]} = (value_{[-1,1]} + 1)/2$ Where $value_{[-1,1]}$ is the normalized value between -1 and 1 and $value_{[0,1]}$ is the normalized value between 0 and 1.

**Description of the step 204 of determining first normalized scores and the step 304 of determining second normalized scores**

The first normalized scores can be obtained by multiplying the first normalized values with the first causality coefficients. The second normalized scores can be obtained by multiplying the second normalized values with the second causality coefficients.

The first and second causality coefficients are obtained using Hill's causality analysis. The Hill's causality analysis allows the obtention of Hill's causality criteria associated respectively with the physiological parameters and the environmental parameters. The higher the Hill's causality criterion is the higher the associated parameter (physiological or environmental) affects the visual fatigue of a patient.

Therefore, the Hill's causality analysis helps to rank the different causes of visual fatigue.

Using the Hill's causality analysis one can obtain first and second causality coefficients in the range of 0 to 16. In this case one can advantageously normalize the first and second causality coefficients to have these coefficients in the range between 0 to 1.

The table below presents example of Hill' causality coefficients and causality coefficients for different parameters.

| Group | Parameter | Hill's causality | Causality coefficient |
|---|---|---|---|
| Refraction | Astigmatism | 16 | 1 |
| | Uncorrected ametropia | 10 | 0.625 |
| | Uncorrected presbyopia | 12 | 0.75 |
| Dry eye-related | Dry eye diagnosis | 14 | 0.875 |
| | Tear film stability (TBT) | 12 | 0.75 |
| | Blink amplitude | 13 | 0.8125 |
| | Blink rate | 9 | 0.5625 |
| | Tear volume | 6 | 0.375 |
| Accommod ation | Amplitude | 6 | 0.375 |
| | Facility | 7 | 0.4375 |
| Light-related | Glare presence | 14 | 0.875 |
| | Blue light presence (e.g., no BL protection) | 15 | 0.9375 |

**Description of the step 205 of selecting at least one of the physiological parameters, and the step 305 of selecting one of the environmental parameters**

The step 205 can be configured to select the physiological parameter associated with the highest normalized score, as the at least one physiological cause of the visual fatigue.

The step 305 can be configured to select the environmental parameter associated with the highest normalized score, as the at least one environmental cause of the visual fatigue.

In other embodiments one can select during step 205 a plurality of the physiological parameters. For example, one can select a predetermined number of the physiological parameters or one can select the physiological parameters having an associated first normalized score above a first predetermined threshold.

Similarly, one can select during step 305 a plurality of the environmental parameters. For example, one can select a predetermined number of the environmental parameters, or one can select the environmental parameters having an associated second normalized score above a second predetermined threshold.

In embodiments two causes of the visual fatigue can be determined. A first cause can be associated with the physiological parameters and a second cause can associated with the environmental parameters.

In other embodiments one can also determine a single cause by comparing the first normalized score of the psychological cause and the second normalized score of the environment cause and by selecting the single cause as the one among the environment cause and a physiological cause with the highest normalized score.

### *Examples of treatments mitigating the visual fatigue causes*

The following table describes for the different visual fatigue causes examples of treatments.

| Visual fatigue causes | Treatments |
|---|---|
| Dry eye | Insulation frames, IR filters, Eye drops |
| Blinking | Blink training, Blink reminders |
| Tear film | IR filters, Insulation frames |
| Uncorrected astigmatism | Precise prescription |
| Uncorrected presbyopia | Near additions, PAL |
| Accommodation problem | Additions |
| Convergence problem | Prisms |
| Blue light | Blue light filters |
| Glare | Anti-glare coating, Peripheral gradient transmission methods, Workstation adjustment, Light adjustment |
| Lighting | Light adjustment, Portable personal lighting |
| Display | Screen lighting and color adjustment, Display content adjustment |
| Posture | Posture monitoring, Posture training |

The posture monitoring, posture training, blink training and blink reminders can be realized using an application implemented for example on the smartphone of the patient.

### *Examples of uses of the system and method of this disclosure*

During the steps 201 and 202 one can obtain value of the following non-exhaustive lists of parameters.

| Group | Measure | Method | Unit |
|---|---|---|---|
| Refraction | Astigmatism | Subjective refraction | Diopter |
| | Uncorrected ametropia | Subjective refraction | Diopter |
| | Uncorrected presbyopia | Subjective refraction | Diopter |
| Dry eye-related | Dry eye diagnosis | Comprehensiv e evaluation | Binary - yes 1 or no 0 |
| | Tear film stability (TBT) | Tear break time | seconds |
| | Blink amplitude | Observation, eye tracking | E.g., percentage of eye closure during blinking |
| | Blink rate | Observation, eye tracking | Blinks per minute |

(continued)

| Group | Measure | Method | Unit |
|---|---|---|---|
| | Tear volume | Shirmers test | milimeters |
| Accommodation | Amplitude | E.g., push up test | Diopter |
| | Facility | Facility test | Cycles per minute |
| Light-related | Glare presence | History, questionnaire | Binary - yes 1 or no 0 Alternatively, proportion of time |
| | Blue light presence (e.g., no BL protection) | History, questionnaire | Binary - yes 1 or no 0 Alternatively, proportion of time |

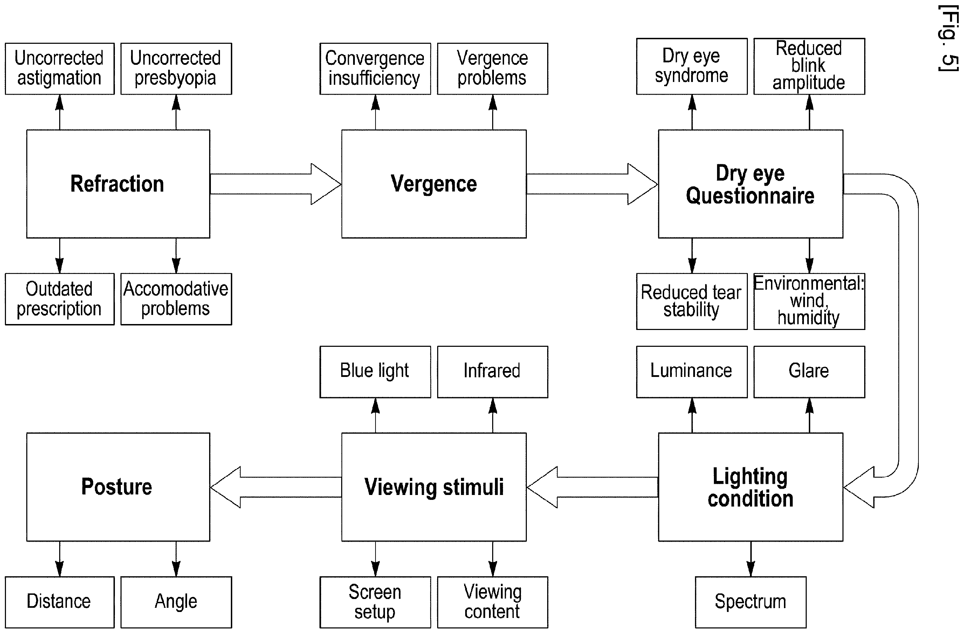

The values can be obtained via measurements or from another device. The order of obtaining these values can take different combinations. Figure 5 represents a specific order of the obtention of the values of the parameters (physiological and environmental).

The table below shows a first example of calculation of the visual fatigue profile of a patient. The normalized value representing the presence/absence (1/0) of a parameter associated with visual fatigue. This normalized value is multiplied by the causality coefficient (normalized Hill's causality) and averaged across each group.

| Group | Parameter | Normalized value (presence of the problem) | Hill's causality | Causality coefficient | Normalized score | Average of scores |
|---|---|---|---|---|---|---|
| **Refraction** | Astigmatism | 1 | 16 | 1 | 1 | 0.583333 333 |
| | Uncorrected ametropia | 0 | 10 | 0.625 | 0 | |
| | Uncorrected presbyopia | 1 | 12 | 0.75 | 0.75 | |
| **Dry eye-related** | Dry eye diagnosis | 1 | 14 | 0.875 | 0.875 | 0.4375 |
| | Tear film stability (TBT) | 1 | 12 | 0.75 | 0.75 | |
| | Blink amplitude | 0 | 13 | 0.8125 | 0 | |
| | Blink rate | 1 | 9 | 0.5625 | 0.5625 | |
| | Tear volume | 0 | 6 | 0.375 | 0 | |
| **Accommodation** | Amplitude | 1 | 6 | 0.375 | 0.375 | 0.1875 |
| | Facility | 0 | 7 | 0.4375 | 0 | |
| **Light-related** | Glare presence | 1 | 14 | 0.875 | 0.875 | 0.4375 |
| | Blue light presence (e.g., no BL protection) | 0 | 15 | 0.9375 | 0 | |

In this example the highest causality coefficient is associated with the presence of astigmatism with a score of 1 indicating that from the parameters evaluated, astigmatism is most likely to cause visual fatigue. For the rest of the parameters listed in the table above, the Hill's causality ranges between and 15 and 7.

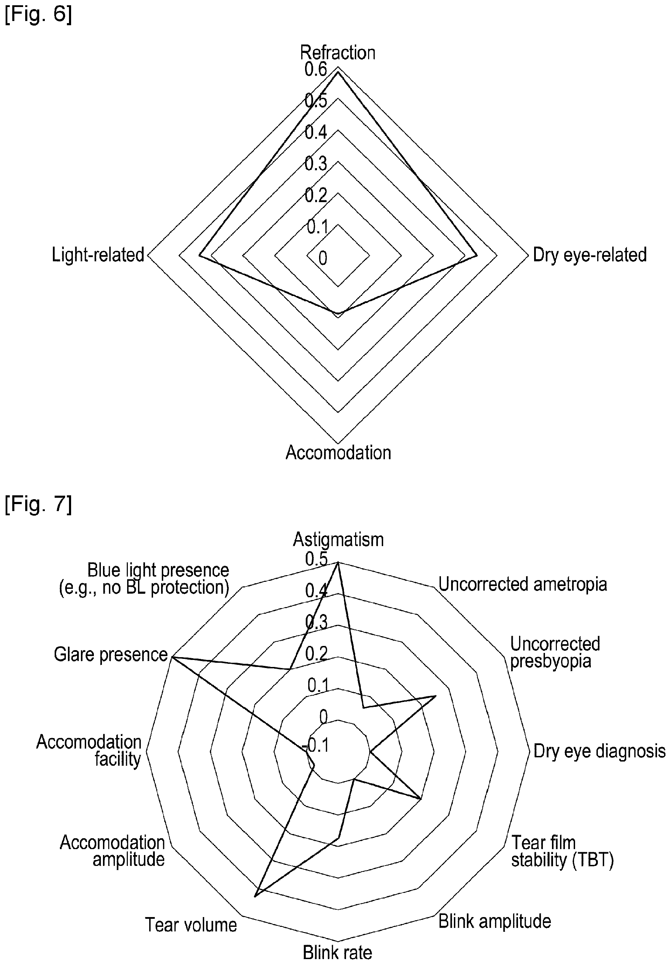

The figure 6 represents the different sums associated respectively with the groups for this first example. For this patient his most likely cause of visual fatigue is related to absence or incorrect refraction in combination with dry eye-related problems and light-related problems. Accordingly, proper refraction and dry eye management followed by advisory is indicated as treatment.

The table below shows a second example of calculation of the visual fatigue profile of a patient. The value of each parameter is normalized based on "Good end" and "Bad end" of the range where "Good end" corresponds to 0 and "Bad end" corresponds to 1. This normalized value is multiplied by the causality coefficient (normalized Hill's causality criterion). In this example, each parameter is considered individually and after that the scores are averaged across each group.

| Group | Parameter | Value | Good end | Bad end | Normalized value | Hill's causality | Causality coefficient | Normalized score | Average of scores |
|---|---|---|---|---|---|---|---|---|---|
| Refraction | Astigmatism | 0.5 | 0 | 1 | 0.5 | 16 | 1 | 0.5 | 0.242 |
| | Uncorrected ametropia | 0.25 | 0 | 4 | 0.0625 | 10 | 0.625 | 0.0391 | |
| | Uncorrected presbyopia | 0.5 | 0 | 2 | 0.25 | 12 | 0.75 | 0.1875 | |
| | Dry eye diagnosis | 0 | 0 | 1 | 0 | 14 | 0.875 | 0 | 0.117 |
| Dry eye-related | Tear film stability (TBT) | 25 | 35 | 10 | 0.4 | 12 | 0.75 | 0.3 | |
| | Blink amplitude | 100 | 100 | 0 | 0 | 13 | 0.8125 | 0 | |
| | Blink rate | 25 | 30 | 1 | 0.172 | 9 | 0.5625 | 0.0970 | |
| | Tear volume | 7 | 10.5 | 3.5 | 0.5 | 6 | 0.375 | 0.1875 | |
| Accommodation | Acommodative amplitude | 50 | 10 | 200 | 0.211 | 6 | 0.375 | 0.0789 | 0.0395 |
| | Acommodative facility | 20 | 20 | 10 | 0 | 7 | 0.4375 | 0 | |
| Light-related | Glare presence | 50 | 0 | 100 | 0.5 | 14 | 0.875 | 0.4375 | 0.3125 |
| | Blue light presence (e.g., no BL protection) | 20 | 0 | 100 | 0.2 | 15 | 0.9375 | 0.1875 | |

The figure 7 represents the different sums associated respectively with the parameters for this second example. For this patient his most likely cause of visual fatigue is related to uncorrected or undercorrected astigmatism in combination with presence of glare and insufficient tear volume. Accordingly, correction of astigmatism, advisory, and dry eye management is indicated as treatment.

**Claims**

1.  Calculation module (102) for determining at least one cause of a visual fatigue of a patient, the calculation module (102) being configured:

    - to obtain values associated respectively with physiological parameters of the patient,
    - to obtain causality coefficients associated respectively with the physiological parameters,
    - to normalize the values to obtain normalized values associated respectively with the physiological parameters,
    - to determine normalized scores associated respectively with the physiological parameters, based on the causality coefficients and the normalized values and,
    - to select at least one of the physiological parameters, using the normalized scores, as the at least one cause of the visual fatigue.

2.  Calculation module (102) according to the claim 1, the calculation module (102) being configured to normalize at least one of the values based on at least one of:

    - a range of possible values of the physiological parameter associated with the at least one of the values,
    - a mean value of the range,
    - a standard deviation of the range,
    - a span of the range,
    - a detection level of the physiological parameter associated with the at least one of the values, and of
    - a value representing a direction of an evolution of the physiological parameter associated with the at least one of the values.

3.  Calculation module (102) according to the claim 2, the calculation module (102) being configured to normalize the at least one of the values by:

    - comparing the at least one of the values with the detection level,
    when the at least one of the values is above the detection level, the normalized value is equal to a first predetermined value,
    when the at least one of the values is below the detection level, the normalized value is equal to a second predetermined value.

4.  Calculation module (102) according to the claim 2 or 3, the calculation module (102) being configured to normalize the at least one of the values by

    - subtracting the mean to the at least one of the values,
    - dividing the subtraction by half the span, to obtain the normalized value.

5.  Calculation module (102) according to any one of the claims 2 to 4, the calculation module (102) being configured to normalize the at least one of the values by

    - subtracting the mean to the at least one of the values,
    - dividing the subtraction by the standard deviation of the range multiplied by a deviation factor to obtain the normalized value.

6.  Calculation module (102) according to any one of the claims 1 to 5, the calculation module (102) being configured:

    - to sum normalized scores associated respectively to a part of the physiological parameters to obtain an aggregated score associated to the part of the physiological parameters,
    - to select the at least one of the physiological parameters, also using the aggregated score.

7. Calculation module (102) according to the claim 6, the calculation module (102) being also configured:
to normalize the aggregated score.

8. Calculation module (102) according to any one of the claims 1 to 7, the physiological parameters being chosen among:

   • parameters related to a visual health of the patient,
   • parameters representing a dryness of an eye of the patient and
   • parameters related to an accommodation capacity of the patient.

9. Calculation module (102) according to any of the claims 1 to 8, the calculation module (102) being configured

   - to select the physiological parameter associated with the highest normalized score, as the at least one cause of the visual fatigue.

10. Calculation module (102) according to any one of the claims 1 to 9,

    the values being first values,
    the causality coefficients being first causality coefficients,
    the normalized values being first normalized values,
    the normalized scores being first normalized scores
    the calculation module (102) being also configured:

       - to obtain second values associated respectively with environmental parameters,
       - to obtain second causality coefficients associated respectively with the environmental parameters,
       - to normalize the second values to obtain second normalized values associated respectively with the environmental parameters
       - to determine second normalized scores associated respectively with the environmental parameters, by multiplying the second causality coefficients and the second normalized values,
       - to select one of the environmental parameters, using the second normalized scores, as an environmental cause of the visual fatigue.

11. Calculation module (102) according to the claim 10, the environmental parameters being chosen among:

    - parameters characteristic of a light received by the patient,
    - a luminance level of the light received by the patient,
    - an amount of UV in the light received by the patient,
    - a contrast between a luminance level of a light originated from an area of interest and a luminance level of an ambient light surrounding the area of interest,
    - parameters characteristic of an air surrounding the patient,
    - a level of a humidity of the air surrounding the patient and
    - parameters characteristic of a pollution of the air surrounding the patient.

12. Calculation module (102) according to any one of the claims 1 to 11, the calculation module (102) being also configured:
    to command a display unit (103) to display the at least one selected physiological parameter and/or the at least one selected environmental parameter.

13. Calculation module (102) according to the claim 12, the calculation module (102) being also configured to command the display unit (103) to display a treatment to mitigate the visual fatigue,
    the treatment depending on the at least one selected physiological parameter and/or the at least one selected environmental parameter.

14. System (101) for determining at least one cause of a visual fatigue of a patient, the system comprising a calculation module (102) for determining at least one cause of a visual fatigue of a patient and a display unit (103),
    the calculation module (102) being configured:

    - to obtain values associated respectively with physiological parameters of the patient,
    - to obtain causality coefficients associated respectively with the physiological parameters,

- to normalize the values to obtain normalized values associated respectively with the physiological parameters,
- to determine normalized scores associated respectively with the physiological parameters, based on the causality coefficients and the normalized values,
- to select at least one of the physiological parameters, using the normalized scores, as the at least one cause of the visual fatigue,
and
- to command the display unit (103) to display the at least one selected physiological parameter.

**15.** Computer implemented method for determining at least one cause of a visual fatigue of a patient, the computer implemented method comprising:

- (201) obtaining values associated respectively with physiological parameters of the patient,
- (202) obtaining causality coefficients associated respectively with the physiological parameters,
- (203) normalizing the values to obtain normalized values associated respectively to the values,
- (204) determining normalized scores associated respectively with the physiological parameters, based on the causality coefficients and the normalized values,
- (205) selecting at least one of the physiological parameters, using the normalized scores, as the at last one cause of the visual fatigue.

[Fig. 1]

[Fig. 2]

[Fig. 3]

[Fig. 4]

[Fig. 5]

[Fig. 6]

[Fig. 7]

Europäisches
Patentamt

European
Patent Office

Office européen
des brevets

# EUROPEAN SEARCH REPORT

**Application Number**

**EP 23 30 5257**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HIRZLE TERESA ET AL: "Understanding, Addressing, and Analysing Digital Eye Strain in Virtual Reality Head-Mounted Displays", ACM TRANSACTIONS ON COMPUTER-HUMAN INTERACTION, ACM, NEW YORK, NY, US, vol. 29, no. 4, 31 March 2022 (2022-03-31), pages 1-80, XP059024132, ISSN: 1073-0516, DOI: 10.1145/3492802 * page 33:4 – page 33:5 * * page 33:8 – page 33:12; table 1 * ----- | 1-15 | INV. G16H50/20 G16H50/30 |
| X | EP 3 113 135 A1 (ESSILOR INT [FR]) 4 January 2017 (2017-01-04) * paragraph [0026] – paragraph [0127] * ----- | 1-15 | |
| X | FENG YONG ET AL: "Investigation of Weighted Scales for Measuring Visual Fatigue in Screening Tasks", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 5768-5771, XP034041616, DOI: 10.1109/EMBC46164.2021.9630334 [retrieved on 2021-11-29] * page 5768 – page 5770 * ----- | 1-15 | TECHNICAL FIELDS SEARCHED (IPC) G16H |

-/--

The present search report has been drawn up for all claims

| Place of search | Date of completion of the search | Examiner |
|---|---|---|
| Munich | 1 August 2023 | Lavin Liermo, Jesus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 23 30 5257

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROSENFIELD MARK: "Computer vision syndrome: a review of ocular causes and potential treatments : Computer vision syndrome", OPHTHALMIC AND PHYSIOLOGICAL OPTICS., vol. 31, no. 5, 12 April 2011 (2011-04-12), pages 502-515, XP093069131, GB ISSN: 0275-5408, DOI: 10.1111/j.1475-1313.2011.00834.x * page 502 - page 511 * ----- | 1-15 | |
| A | XIAO-WEI SHI ET AL: "Visual fatigue diversity research of static and dynamic lighting environment", INDUSTRIAL ENGINEERING AND ENGINEERING MANAGEMENT (IE&EM), 2011 IEEE 18TH INTERNATIONAL CONFERENCE ON, IEEE, 3 September 2011 (2011-09-03), pages 291-294, XP032056014, DOI: 10.1109/ICIEEM.2011.6035160 ISBN: 978-1-61284-446-6 * page 291 - page 293 * ----- | 1-15 | TECHNICAL FIELDS SEARCHED (IPC) |

The present search report has been drawn up for all claims

| Place of search | Date of completion of the search | Examiner |
|---|---|---|
| Munich | 1 August 2023 | Lavin Liermo, Jesus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5257

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2023

| Patent document cited in search report | | Publication date | Patent family member(s) | | Publication date |
|---|---|---|---|---|---|
| EP 3113135 | A1 | 04-01-2017 | EP 3113135 | A1 | 04-01-2017 |
| | | | WO 2017001319 | A1 | 05-01-2017 |

EPO FORM P0459

For more details about this annex : see Official Journal of the European Patent Office, No. 12/82